# EUROPÄISCHE PATENTSCHRIFT

(11) **EP 2 183 238 B1**
(45) Veröffentlichungstag und Bekanntmachung des Hinweises auf die Patenterteilung: **02.03.2011**
(21) Anmeldenummer: 08786186.0
(22) Anmeldetag: 16.07.2008
(51) Int. Cl.: C07D 317/20, C07D 319/06, C10L 10/00

(54) **VERFAHREN ZUR HERSTELLUNG VON ZYKLISCHEN GLYZERINACETALEN ODER ZYKLISCHEN GLYZERINKETALEN ODER GEMISCHE DERSELBEN**
PROCESS FOR PREPARING CYCLIC GLYCEROL ACETALS OR CYCLIC GLYCEROL KETALS OR MIXTURES THEREOF
PROCÉDÉ DE FABRICATION D'ACÉTALS CYCLIQUES DE GLYCÉRINE OU DE CÉTALS CYCLIQUES DE GLYCÉRINE OU DE LEURS MÉLANGES

(30) Priorität: 17.07.2007 AT 11252007
(43) Veröffentlichungstag der Anmeldung: 12.05.2010
(73) Patentinhaber: Christof International Management Gmbh, 8010 Graz (AT)
(72) Erfinder: WIMMER, Theodor, A-8081 Heiligenkreuz (AT); ERTL, Christof, A-4755 Zell/Pram (AT); KALB, Roland, A-8700 Leoben (AT)
(74) Vertreter: Babeluk, Michael
(86) Internationale Anmeldenummer: PCT/EP2008/059293
(87) Internationale Veröffentlichungsnummer: WO 2009/010527

(56) Entgegenhaltungen:
- EP-A- 1 331 260
- WO-A-2005/093015
- DA YAN HE ET AL: "STUDIES ON CARBOHYDRATES X A NEW METHOD FOR THE PREPARATION OF ISOPROPYLIDENE SACCHARIDES" SYNTHETIC COMMUNICATIONS, TAYLOR & FRANCIS, PHILADELPHIA, PA, Bd. 22, Nr. 18, 1. Januar 1992 (1992-01-01), Seiten 2653-2658, XP001029518 ISSN: 0039-7911
- GORLA ET AL.: "Cationic Palladium(II), and Rhodium(I) Complexes as Acetalisation Catalysts" HELVETICA CHIMICA ACTA, Bd. 73, Nr. 3, 1990, Seiten 690-697, XP002499566

## Beschreibung

Die Erfindung betrifft ein Verfahren zur Herstellung von zyklischen Glyzerinacetalen oder zyklischen Glyzerinketalen oder Gemische derselben und zyklischen Glyzerinmonoester- oder monoetheracetalen oder zyklischen Glyzerinmonoester- oder monoetherketalen oder Gemischen derselben durch Umsetzung von Glyzerin bzw. dessen Monoestern oder Monoethern mit Aldehyden oder Ketonen in Gegenwart eines sauren heterogenen Katalysators.

Glyzerin fällt unter anderem bei der Alkoholyse von Fettsäureglyzeriden mit niederen einwertigen Alkoholen insbesondere mit Methanol zu Fettsäuremethylestern als Nebenprodukt an.

Diese Reaktion hat in letzter Zeit große Bedeutung zur Herstellung von Biodiesel erlangt und wird in großtechnischem Maßstab durchgeführt, wobei große Mengen Glyzerin anfallen, die am herkömmlichen Markt für Glyzerin nicht mehr unterzubringen sind, was zu einem starken Preisverfall geführt hat.

Glyzerin selbst ist als Motorentreibstoff infolge seiner chemischen und physikalischen Eigenschaften nicht geeignet, insbesondere wegen seiner Unlöslichkeit in Dieseltreibstoffen, seiner hohen Viskosität und seines hohen Siedepunktes.

Es wurde daher schon mehrfach versucht, das Glyzerin in Derivate überzuführen, die in Diesel, Biodiesel oder Ottokraftstoffen löslich sind, eine niedrige Viskosität und einen Siedepunkt aufweisen, der im Bereich der herkömmlichen Treibstoffe liegt.

Eine Gruppe derartiger Derivate sind zyklische Acetale und Ketale des Glyzerins aus der Reaktion von Glyzerin mit Aldehyden mit 1 bis 6 Kohlenstoffen und /oder Ketonen mit einer Kettenlänge von 3 bis 10 Kohlenstoffatomen.

So wird beispielsweise in der EP 1 331 260 vorgeschlagen, das bei der Umesterung von Triglyceriden mit Methanol oder Ethanol anfallende Glyzerin mit Aldehyden oder Ketonen umzusetzen und die erhaltenen Glyzerinacetale und Ketale den Fettsäuremethylestern zuzusetzen.

Die Glyzerinacetale und Ketale können aber auch als wertvolle Lösungsmittel oder als Zwischenprodukte zur Herstellung weiterer Derivate des Glyzerins zur Verwendung als Treibstoffadditive verwendet werden.

Die Herstellung von Glyzerinacetalen und Ketalen ist schon lange bekannt. Sie erfolgt durch säuekatalysierte Umsetzung von Glyzerin mit Aldehyden wie z.B. Formaldehyd, Acetaldehyd, oder Butyraldehyd und Ketonen wie Aceton oder Ethyl-methyl-keton in Anwesenheit von Chlorwasserstoff (E. Fischer, B.dtsch.ch.Ges., 53 (1920) S.1027) oder p-Toluol-sulfonsäure (Newman/Renoll, J.Am.Chem.Soc., 67 (1945) S.1621).

Die Bildung eines Acetals aus der Reaktion eines Aldehyds mit einem Alkanol lässt sich wie folgt darstellen:

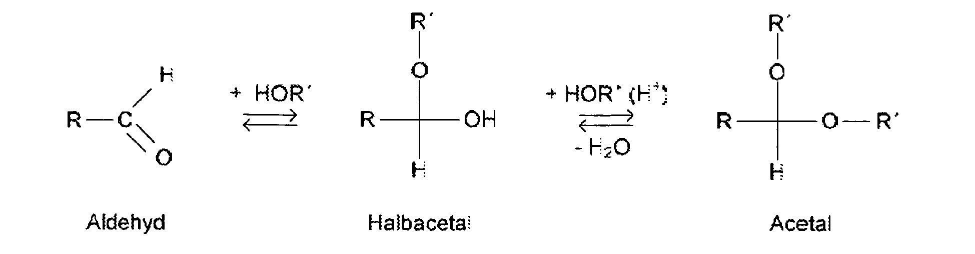

Die Bildungsreaktion eines Ketals verläuft analog unter Verwendung eines Ketons. Bei der Bildung eines zyklischen Acetals bzw. Ketals unter Verwendung von Glyzerin erfolgt die Reaktion des Halbacetals bzw. -ketals mit einer zweiten Hydroxylgruppe des Glyzerins.

Zur Verbesserung der Ausbeute wird das Reaktionswasser entweder durch Zugabe von Trockenmittel oder durch azeotrope Destillation mit Schleppmitteln entfernt. Besonders beim Einsatz von niedrig siedenden Aldehyden oder Ketonen sind entsprechend niedrig siedende Schleppmittel wie z.B. Dichlormethan, Chlorform oder niedrigsiedende Petroleumbenzine erforderlich, was lange Reaktionszeiten, den Verbrauch oder die Wiedergewinnung großer Mengen dieser Lösungsmittel zur Folge hat.

Keine dieser Methoden ist daher zur großtechnischen Herstellung geeignet.

Die DE 196 48 960 schlägt daher vor, Glyzerin mit einem Überschuss eines Aldehyds oder Ketons in Gegenwart eines sauren homogenen Katalysators umzusetzen und während der Umsetzung einen Teil des überschüssigen Aldehyds oder Ketons zusammen mit dem Reaktionswasser abzudestillieren und gleichzeitig durch trockenen Aldehyd oder Keton zu ergänzen. Nachteile dieses Verfahrens sind, dass ebenfalls große Überschussmengen Aldehyd oder Keton aufgearbeitet bzw. getrocknet werden müssen.

In der DD 238232 wird vorgeschlagen, Glyzerin und Aceton in Gegenwart eines homogenen sauren Katalysators z.B. Toluolsulfonsäure bei Temperaturen vorzugsweise zwischen 20°C und 50°C umzusetzen, anschließend den Katalysator mit einem basischen Medium zu neutralisieren und die Reaktionsmischung destillativ aufzuarbeiten.

Ein Nachteil dieses Verfahrens ist, dass bezogen auf Glyzerin nur eine maximale Ausbeute von 45% erzielt wird, wobei durch Rückführung des nicht umgesetzten Glyzerins eine Totalausbeute von ca. 95% erzielt wird. Ein weiterer Nachteil ist die Tatsache, dass der saure Katalysator vor der destillativen Aufarbeitung neutralisiert werden muss, was einen Verlust des Katalysators bedeutet, und vor allem ein Problem für die Rückführung des nicht umgesetzten Glyzerins darstellt, in welchen sich der neutralisierte Katalysator befindet.

Eine weitere Schwierigkeit besteht in der Tatsache, dass Glyzerin mit niedrig siedenden Aldehyden und Ketonen nicht mischbar ist. In der DD 238233 wird daher vorgeschlagen, zu den Reaktionskomponenten Glyzerin und Aceton soviel des Endproduktes (Isopropyliden-Glyzerin, IPG) oder bereits reagierter Reaktionsmischung zuzugeben, dass eine homogene Lösung entsteht und diese durch Zugabe eines sauren Ionenaustauschers bei Temperaturen zwischen 35°C und 55°C zur Reaktion bringt und nach Abtrennen des Katalysators das Reaktionsgemisch destillativ auftrennt.

Neben dem Vorteil, dass ein wieder verwendbarer heterogener Katalysator zum Einsatz kommt und das nicht umgesetzte Glyzerin nicht durch einen homogenen Katalysator belastet wird, hat das Verfahren den Nachteil, dass der Umsatz bezogen auf Glyzerin nur etwa 55% beträgt und je Volumenteil Glyzerin 3 Volumenteile Isopropyliden-Glyzerin zurückgeführt und daher 2-fach aufgearbeitet werden müssen.

Die WO 2005/093015 offenbart unter anderem ein Verfahren zur Herstellung von Glyzerinacetalen durch Umsetzung von aus der Herstellung von Biodiesel direkt oder indirekt erhaltene Glyzerin mit Aldehyden und Ketonen bei erhöhter Temperatur und gegebenenfalls erhöhtem Druck. Die Ausbeute an Glyzerinacetal beträgt hierbei unter 80%.

Es ist daher Aufgabe der vorliegenden Erfindung ein kostengünstiges Verfahren zur Herstellung von Acetalen und Ketalen des Glyzerins oder dessen Monoester und Monoether zur Verfügung zu stellen, dass die oben genannten Nachteile überwindet und höhere Ausbeuten als im bekannten Stand der Technik liefert.

Diese Aufgabe wird durch das eingangs erwähnte Verfahren erfindungsgemäß dadurch gelöst, dass die Reaktionskomponenten durch intensives Mischen bei einer Temperatur von 10°C bis 30°C zur Reaktion gebracht werden.

Die Anmelderin hat überraschenderweise festgestellt, dass das Reaktionsgleichgewicht umso weiter auf der Seite des erwünschten Reaktionsproduktes liegt, je tiefer die Reaktionstemperatur ist. So werden bei der Aufarbeitung des Reaktionsgemisches nach Abtrennen des Katalysators bei 20°C Umsätze an Glyzerin von 90% erhalten.

Weiters wurde gefunden, dass das anfänglich inhomogene Gemisch der Reaktionskomponenten bei einer Temperatur von 20°C innerhalb von 3 bis 15 Minuten zu einer ausreichend homogenen Reaktionslösung führt - wobei das Gleichgewicht bei 90% umgesetztem Glyzerin liegt - wenn die Reaktionskomponenten und der Katalysator einer intensiven Mischung bzw. Turbulenzbildung unterzogen werden. Somit ist im Gegensatz zum bekannten Stand der Technik eine Rückführung von Reaktionsprodukt zur Homogenisierung der Reaktionsmischung nicht notwendig, wodurch wiederum die Ausbeute gesteigert wird. Die intensive Durchmischung der Reaktionspartner fördert die Bildung einer Emulsion mit geringer Tröpfchengröße. Dadurch wird in Verbindung mit dem heterogenen Katalysator eine sehr große Reaktionsoberfläche erzielt, wodurch die erfindungsgemäßen hohen Glyzerinumsätze erhalten werden. Ein wesentlicher Vorteil des erfindungsgemäßen Verfahrens ist, dass die Reaktionszeiten nur einen Bruchteil der im Stand der Technik bekannt gewordenen Verfahren betragen. Dadurch werden bei der Herstellung von Acetalen und Ketalen hohe Durchsätze erzielt.

Das erfindungsgemäße Verfahren liefert besonders gute Ausbeuten, wenn ein Aldehyd mit 1 bis 10 Kohlenstoffatomen, insbesondere Formaldehyd, Acetaldehyd, Propionaldehyd, n- oder i-Butyraldehyd, 3-Methylbutanal-(1) oder 2-Methylbutanal-(1) oder ein Keton mit 3 bis 10 Kohlenstoffatomen, insbesondere Aceton oder Methylketon, oder ein Gemisch derselben eingesetzt wird.

Des Weiteren kann das erfindungsgemäße Verfahren bei Normaldruck durchgeführt werden. Dies hat den Vorteil, dass der Reaktor, in dem die Reaktion durchgeführt wird, einen einfachen Aufbau aufweist und insbesondere keine kostenintensiven Maßnahmen für eine erhöhte Druckfestigkeit des Reaktors erforderlich sind.

Um eine hohe Ausbeute zu erzielen, werden bevorzugterweise die Aldehyde oder Ketone oder die Gemische derselben in einem bis zu 10fachen Überschuss eingesetzt, wobei der Überschuss an Reaktanten nach erfolgter Umsetzung des Glyzerins zum Beispiel destillativ abgetrennt wird und für weitere Glyzerinumsetzungen wiederverwendet werden kann.

In einer bevorzugten Variante der Erfindung ist der heterogene Katalysator ein stark saures Ionenaustauscherharz, ein stark saurer flüssiger Ionenaustauscher, ein Zeolith, ein Molekularsieb oder ein saures Aluminiumoxid. Diese Katalysatoren weisen im Allgemeinen eine große Oberfläche auf und lassen sich nach erfolgter Reaktion leicht durch beispielsweise Filtrieren von dem Reaktionsgemisch abtrennen. Flüssige Katalysatoren lassen sich mittels Destillation oder auch mittels Membranverfahren oder adsorptive und extraktive Methoden aus dem Reaktionsprodukt entfernen.

Alternativ zu dem beispielsweise bei der Herstellung von Biodiesel anfallendem Glyzerin können in dem erfindungsgemäßen Verfahren auch Glyzerinderivate, insbesondere Glyzerinmonocarbonsäureester Glyzerinmonoacetat, -monopropionat, und Gylcerinmonocarbonsäureester mit 5 bis 21 Kohlenstoffatomen oder als Glyzerinmonoether Glyzerinmonomethylether, Glyzerinmonoethylether, Glyzerinmono-n-propyl- oder Glyzerinmono-isopropylether oder Glyzerinmono-n-butyl-, Glyzerinmono-isobutyl- oder Glyzerinmono-tertiärbutylether oder Gemische derselben eingesetzt werden.

Ein weiterer Vorteil des erfindungsgemäßen Verfahrens ist, dass es in Gegenwart eines sauren heterogenen Katalysators wie z.B. eines stark sauren Ionenaustauscherharzes, eines stark sauren flüssigen Ionenaustauschers, eines Zeolithes, eines Molekularsiebes oder eines sauren Aluminiumoxides sowohl diskontinuierlich als auch semikontinuierlich oder kontinuierlich durchgeführt werden kann, wobei das Reaktionsgemisch nach Abtrennen des Katalysators destillativ aufgearbeitet wird und die aus der Reaktionsmischung destillativ abgetrennten Ausgangsstoffe zur Reaktion wieder zurückgeführt werden. Gegebenenfalls kann der Katalysator durch Behandlung mit wässriger oder wässrig-alkoholischer mineralischer oder organischer Säure regeneriert und wiederverwendet werden.

Ein zum Einsatz kommender Festbettkatalysator kann beispielsweise aus regelmäßig oder unregelmäßig geformten, extrudierten Granulaten, Kügelchen oder Brocken mit einem Durchmesser von 0,5 mm bis 10 mm in loser Schüttung bestehen oder aber auf einem festen Träger aufgebracht sein. Er kann auch in Form eines porösen Materials vorliegen, durch welches das Reaktionsmedium hindurchströmt.

Vorzugsweise wird die Reaktion kontinuierlich ausgeführt, wobei Glyzerin und ein Aldehyd mit einer Kettenlänge von 1 bis 10 Kohlenstoffatomen oder ein Keton mit einer Kettenlänge von 3 bis 10 Kohlenstoffatomen oder ein Gemisch derselben in einem Molverhältnis 1 zu 1 bis 1 zu 10, besonders bevorzugt in einem Molverhältnis 1 zu 2 bis 1 zu 6 mittels Dosierpumpen einem mit einem Katalysator, bevorzugterweise sauren Ionenaustauscherharz befüllten kontinuierlich arbeitenden Reaktor, - z.B. einem Rohrreaktor,- bei einer Temperatur zwischen 0°C und 50°C, vorzugsweise zwischen 10°C und 30°C zugeführt werden.

Die Strömungsgeschwindigkeiten der Reaktanten sollten derart gewählt werden, dass die zur Überwindung der Grenzflächenspannung benötigte Arbeit in das Reaktionsgemisch eingebracht wird. Die so entstehenden Turbulenzen erlauben die Bildung einer Emulsion, wodurch eine erhöhte Reaktionsoberfläche erhalten wird, die wiederum Voraussetzung für einen hohen Reaktionsumsatz bereits bei niedrigen Temperaturen unter Verwendung eines heterogenen Katalysators ist. Es handelt sich bei dem erfindungsgemäßen Verfahren um eine Dreiphasen-Grenzflächenreaktion (flüssig/flüssig/fest). Die Zufuhr der Reaktanten erfolgt mit einer derart gewählten Geschwindigkeit und Druck, dass die Verweilzeit im Rohrreaktor zwischen 0,5 und 30 Minuten, vorzugsweise zwischen 2 und 15 Minuten beträgt. Die Verweilzeit und damit die Strömungsgeschwindigkeit sind insbesondere auch von dem Reaktordesign (z.B. Verhältnis von Länge zu Durchmesser des Reaktors) abhängig. Die entstehenden Turbulenzen im Katalysatorbett reichen bei entsprechender Strömungsgeschwindigkeit aus, um die Komponenten aufgrund der Emulsionsbildung und der damit verbundenen erhöhten Reaktionsoberfläche bereits bei geringer Temperatur und bevorzugterweise bei Normaldruck zur Reaktion zu bringen. Die Turbulenzen entstehen aufgrund der Strömungsgeschwindigkeit der Reaktanten am Festbettkatalysator; zusätzlich können weitere bauliche Maßnahmen wie Prallbleche, Rippen, Füllkörper oder andere strömungsbrechende Konstruktionen eingesetzt werden. Diese Maßnahmen haben sich besonders bei dem Einsatz eines flüssigen, mit den Reaktanten nicht-mischbaren Katalysators bewährt.

Bei einem diskontinuierlichen Verfahren können im Reaktor zusätzlich Misch- und Rühreinrichtungen vorgesehen sein.

In einer bevorzugten Variante der Erfindung werden die Reaktionskomponenten vor dem Eintritt in den Reaktor durch eine statische oder dynamische Mischeinrichtung vorgemischt bzw. emulgiert und hernach in den Reaktor gepumpt. Dadurch werden gleichbleibend Emulsionen mit denselben Charakteristika erhalten, wodurch wiederum die Ausbeute an umgesetztem Glyzerin in den unterschiedlichen Arbeitszyklen des Reaktors weniger stark variiert.

Bevorzugterweise wird bei dem erfindungsgemäßen Verfahren das bei der Herstellung von Biokraftstoffen für Verbrennungsmotoren anfallende Glyzerin als Reaktionskomponente eingesetzt. Das hierbei zum Einsatz kommende Glyzerin kann gereinigt oder roh verwendet werden.

Die nach dem oben beschriebenen Verfahren hergestellten Produkte, insbesondere zyklische Glyzerinacetale oder zyklischen Glyzerinketale oder Gemische derselben und zyklischen Glyzerinmonoester- oder monoetheracetalen oder zyklischen Glyzerinmonoester- oder monoetherketalen oder Gemischen derselben, werden bevorzugterweise als Treibstoffadditive oder als Ausgangsstoffe für die Herstellung derselben eingesetzt.

Die Erfindung wird durch die folgenden Beispiele in nicht-einschränkender Weise näher erläutert:

### Beispiel 1

Glyzerin und Aceton werden im Molverhältnis von 1 zu 4 mittels Dosierpumpen bei einer Temperatur von 20°C einem mit Dowex DR 2030, einem sauren Ionentauscherharz befüllten Rohrreaktor zugeführt, wobei der Rohrreaktor so dimensioniert ist, dass die Verweildauer etwa 15 Minuten beträgt. Das austretende homogene Reaktionsgemisch wird nun durch Normaldruckdestillation von überschüssigem Aceton und Reaktionswasser befreit und anschließend bei vermindertem Druck das Isopropyliden-Glyzerin (IPG) abdestilliert. Der Siedepunkt des IPG bei 80 mbar beträgt 120°C - 122°C. Das im Destillationsrückstand verbleibende nicht umgesetzte Glyzerin wird zum Rohrreaktor rückgeführt. Das reaktionswasserenthaltende Aceton wird durch Rektifikation getrocknet und ebenfalls zum Rohrreaktor rückgeführt. Bei stetiger Rückführung des nicht umgesetzten Glyzerins beträgt die Ausbeute 95% bezogen auf den Glyzerineinsatz.

### Beispiel 2

Glyzerin und n-Butyraldehyd werden im Molverhältnis 1 zu 2 wie in Beispiel 1 beschrieben bei einer Temperatur von 20°C einem mit saurem Ionenaustauscher befüllten Rohrreaktor zugeführt und nach ca. 15 Minuten Verweildauer destillativ aufgearbeitet. Der überschüssige n-Butyraldehyd und das Reaktionswasser destillieren dabei als zweiphasiges Gemisch ab, wobei der als leichtere Phase abgetrennte n-Butyraldehyd ohne weitere Behandlung wiederum dem Rohrreaktor zugeführt werden kann. Nach einer Druckreduktion auf 80 mbar destilliert n-Butyliden-Glyzerin bei 134°C - 138°C ab. Die Ausbeute bei stetiger Rückführung des nicht umgesetzten Glyzerins beträgt 97% bezogen auf den Glyzerineinsatz.

## Patentansprüche

1. Verfahren zur Herstellung von zyklischen Glyzerinacetalen oder zyklischen Glyzerinketalen oder Gemische derselben und zyklischen Glyzerinmonoester- oder monoetheracetalen oder zyklischen Glyzerinmonoester- oder monoetherketalen oder Gemischen derselben durch Umsetzung von Glyzerin bzw. dessen Monoestern oder Monoethern mit Aldehyden oder Ketonen in Gegenwart eines sauren heterogenen Katalysators, **dadurch gekennzeichnet, dass** die Reaktionskomponenten durch intensives Mischen bei einer Temperatur von 10°C bis 30°C zur Reaktion gebracht werden.

2. Verfahren nach Anspruch 1, **dadurch gekennzeichnet, dass** ein Aldehyd mit 1 bis 10 Kohlenstoffatomen oder ein Keton mit 3 bis 10 Kohlenstoffatomen oder ein Gemisch derselben eingesetzt wird.

3. Verfahren nach einem der Ansprüche 1 oder 2, **dadurch gekennzeichnet, dass** als Aldehyd Formaldehyd, Acetaldehyd, Propionaldehyd, n- oder i-Butyraldehyd, 3-Methylbutanal-(1) oder 2-Methylbutanal-(1) und als Ketone Aceton oder Methylketon zum Einsatz kommen.

4. Verfahren nach einem der Ansprüche 1 bis 3, **dadurch gekennzeichnet, dass** die Aldehyde oder Ketone oder die Gemische derselben in einem bis zu 10fachen Überschuss eingesetzt werden.

5. Verfahren nach einem der Ansprüche 1 bis 4, **dadurch gekennzeichnet, dass** der heterogene Katalysator ein stark saures Ionenaustauscherharz, ein stark saurer flüssiger Ionenaustauscher, ein Zeolith, ein Molekularsieb oder ein saures Aluminiumoxid ist.

6. Verfahren nach einem der Ansprüche 1 bis 5, **dadurch gekennzeichnet, dass** als Glyzerinmonocarbonsäureester Glyzerinmonoacetat, Glyzerinmonopropionat und Glyzerinmonocarbonsäureester mit 5 bis 21 Kohlenstoffatomen oder als Glyzerinmonoether Glyzerinmonomethylether, Glyzerinmonoethylether, Glyzerinmono-n-propyl- oder -isopropylether oder Glyzerinmono-n-butyl-, -isobutyl- oder -tertiärbutylether oder Gemische derselben eingesetzt werden.

7. Verfahren nach einem der Ansprüche 1 bis 6, **dadurch gekennzeichnet, dass** die Reaktion diskontinuierlich erfolgt und das Reaktionsgemisch nach Abtrennen des Katalysators destillativ aufgearbeitet wird.

8. Verfahren nach einem der Ansprüche 1 bis 6, **dadurch gekennzeichnet, dass** die Reaktion semikontinuierlich oder kontinuierlich erfolgt und das Reaktionsgemisch destillativ aufgearbeitet wird.

9. Verfahren nach einem der Ansprüche 7 oder 8, **dadurch gekennzeichnet, dass** die aus der Reaktionsmischung destillativ abgetrennten Ausgangsstoffe zur Reaktion wieder zurückgeführt werden.

10. Verfahren nach Anspruch 8 oder 9, **dadurch gekennzeichnet, dass** die Reaktionskomponenten kontinuierlich einem mit dem heterogenen Katalysator befüllten Rohrreaktor zugeführt werden und infolge starker Turbulenzen im Katalysatorbett eine intensive Mischung erfolgt.

11. Verfahren nach einem der Ansprüche 7 bis 10, **dadurch gekennzeichnet, dass** die Reaktionskomponenten vor dem Eintritt in den Reaktor durch eine statische oder dynamische Mischeinrichtung vorgemischt bzw. emulgiert werden.

12. Verfahren nach einem der Ansprüche 1 bis 11, **dadurch gekennzeichnet, dass** das bei der Herstellung von Biokraftstoffen für Verbrennungsmotoren anfallende Glyzerin als Reaktionskomponente eingesetzt wird.

## Claims

1. Method for preparing cyclic glycerol acetals or cyclic glycerol ketals or mixtures thereof and cyclic glycerol monoester or monoether acetals or cyclic glycerol monoester or monoether ketals or mixtures thereof, by reacting glycerol or its monoesters or monoethers with aldehyds or ketones in the presence of an acidic, heterogeneous catalyst, **characterised in that** the components are brought to reaction by intensive mixing at a temperature of between 10°C and 30°C.

2. Method according to claim 1, **characterised in that** an aldehyde with 1 to 10 carbon atoms or a ketone with 3 to 10 carbon atoms or a mixture thereof is used.

3. Method according to claim 1 or 2, **characterised in that** formaldehyde, acetaldehyde, propionaldehyde, n- or i-butyraldehyde, 3-methylbutanal-(1) or 2-methylbutanal-(1) is used as the aldehyde and acetone or methyl ketone is used as the ketone.

4. Method according to any of claims 1 to 3, **characterised in that** the aldehydes or ketones or their mixtures are used in up to tenfold excess.

5. Method according to any of claims 1 to 4, **characterised in that** the heterogeneous catalyst is a strongly acidic ion exchanger resin, a strongly acidic liquid ion exchanger, a zeolite, a molecular sieve, or an acidic aluminium oxide.

6. Method according to any of claims 1 to 5, **characterised in that** there are used as glycerol monocarboxylic acid esters glycerol monoacetate, glycerol monopropionate and glycerol monocarboxylic acid esters with 5 to 21 carbon atoms, or as glycerol monoethers glycerol monomethyl ether, glycerol monoethyl ether, glycerol mono-n-propyl or -isopropyl ether or glycerol mono-n-butyl-, -isobutyl- or -tertiary butyl ether or mixtures thereof.

7. Method according to any of claims 1 to 6, **characterised in that** the reaction is carried out discontinuously and the reaction mixture is refined by distillation after removal of the catalyst.

8. Method according to any of claims 1 to 6, **characterised in that** the reaction is carried out semi-continuously or continuously and the reaction mixture is refined by distillation.

9. Method according to any of claims 7 or 8, **characterised in that** the reactants separated from the reaction mixture by distillation are recycled to the reaction.

10. Method according to any of claims 8 or 9, **characterised in that** the components of the reaction are continuously fed into a tubular reactor filled with the heterogeneous catalyst and intensive mixing is achieved due to strong turbulences in the catalyst bed.

11. Method according to any of claims 7 to 10, **characterised in that** the components of the reaction are premixed or emulgated prior to entering the reactor in a static or dynamical mixing unit.

12. Method according to any of claims 1 to 11, **characterised in that** glycerol obtained during the production of biofuels for internal combustion engines is used as a reaction component.

## Revendications

1. Procédé d'obtention d'acétals de glycérine cycliques ou de cétals de glycérine cycliques ou de mélanges de ces composés et de monoester- ou de monoéther acétals de glycérine cycliques ou de monoester- ou monoéther cétals de glycérine cycliques ou de mélanges de ces composés par réaction de glycérine ou de ses monoesters ou monoéthers avec des aldéhydes ou des cétones en présence d'un catalyseur hétérogène acide,
procédé **caractérisé en ce qu'**
on met les composants réactionnels en réaction par mélange intensif à une température de 10°C à 30°C.

2. Procédé conforme à la revendication 1,
**caractérisé en ce qu'**
on met en oeuvre un aldéhyde ayant entre 1 et 10 atomes de carbone ou une cétone ayant entre 3 et 10 atomes de carbone ou un mélange de ces composés.

3. Procédé conforme à l'une des revendications 1 et 2,
**caractérisé en ce qu'**
en tant qu'aldéhyde, on utilise du formaldéhyde, de l'acétaldéhyde, du propionaldéhyde, du n- ou i-butyraldéhyde, du 3-méthylbutanal-1 ou du 2-méthylbutanal-1, et en tant que cétone, on utilise de l'acétone ou de la méthylcétone.

4. Procédé conforme à l'une des revendications 1 à 3,
**caractérisé en ce qu'**
on met en oeuvre l'aldéhyde ou la cétone ou leurs mélanges en un excès allant jusqu'à 10 fois.

5. Procédé conforme à l'une des revendications 1 à 4,
**caractérisé en ce que**
le catalyseur hétérogène est une résine échangeuse d'ions fortement acide, un échangeur d'ions liquide fortement acide, une zéolithe, un tamis moléculaire ou un oxyde d'aluminium acide.

6. Procédé conforme à l'une des revendications 1 à 5,
**caractérisé en ce qu'**
en tant qu'ester d'acide monocarboxylique et de glycérine on met en oeuvre du monoacétate de glycérine, du monopropionate de glycérine, et un ester d'acide monocarboxylique et de glycérine ayant de 5 à 21 atomes de carbone, ou en tant que monoéther de glycérine on met en oeuvre, du monométhyléther de glycérine, du monoéthyléther de glycérine, du mono-n-propyl ou isopropyléther de glycérine, ou du mono-n-butyli-, isobutyli- ou tertbutyléther de glycérine ou un mélange de ces composés.

7. Procédé conforme à l'une des revendications 1 à 6,
**caractérisé en ce qu'**
on met en oeuvre la réaction de manière discontinue et on traite le mélange réactionnel par distillation après séparation du catalyseur.

8. Procédé conforme à l'une des revendications 1 à 6,
**caractérisé en ce qu'**
on met en oeuvre la réaction de manière semi-continue ou continue et on traite le mélange réactionnel par distillation.

9. Procédé conforme à l'une des revendications 7 ou 8,
**caractérisé en ce qu'**
on remet les substances séparées du mélange réactionnel par distillation en réaction.

10. Procédé conforme à l'une des revendications 8 ou 9
**caractérisé en ce qu'**
on transfère les composants réactionnels en continu dans un réacteur tubulaire rempli du catalyseur hétérogène et on effectue un mélange intensif dans le lit catalytique en raison de la présence de fortes turbulences.

11. Procédé conforme à l'une des revendications 7 à 10,
**caractérisé en ce qu'**
avant leur entrée dans le réacteur, on effectue un prémélange ou une mise en émulsion des composants réactionnels dans une installation de mélange statique ou dynamique.

12. Procédé conforme à l'une des revendications 1 à 11,
**caractérisé en ce que**
on met en oeuvre la glycérine obtenue lors de la fabrication de biocarburants pour des moteurs à combustion interne, en tant que composant réactionnel.
